# FASCICULE DE BREVET EUROPEEN

(11) **EP 0 544 588 B1**
(45) Date de publication et mention de la délivrance du brevet: **31.01.1996**
(21) Numéro de dépôt: 92403167.7
(22) Date de dépôt: 25.11.1992
(51) Int. Cl.: C07C 403/06, C07C 403/14, C07C 403/16, C07C 403/22, C07C 391/02, C07C 47/24, C07C 49/227, C07C 45/51

(54) **Nouveaux intermédiaires de préparation des vitamines A et E et des caroténoides**
Zwischenprodukte für die Herstellung von Vitamin A und E und von Carotenoiden
Intermediates for the preparation of vitamin A and E and of carotenoids

(30) Priorité: 28.11.1991 FR 9114699
(43) Date de publication de la demande: 02.06.1993
(73) Titulaire: RHONE-POULENC NUTRITION ANIMALE, F-03600 Commentry (FR)
(72) Inventeur: Ancel, Jean-Erick, F-76000 Rouen (FR); Bienayme, Hugues, F-69002 Lyon (FR); Duhamel, Lucette, F-76130 Mont-Saint-Aignan (FR); Duhamel, Pierre, F-76130 Mont-Saint-Aignan (FR)
(74) Mandataire: Le Pennec, Magali

(56) Documents cités:
- EP-A- 0 314 575
- DE-B- 1 157 606
- US-A- 2 451 739
- US-A- 3 493 619
- US-A- 3 671 575

## Description

La présente invention concerne un nouveau procédé de préparation de la vitamine A et de ses intermédiaires, elle concerne aussi un nouveau procédé de préparation des intermédiaires de la vitamine E et des caroténoïdes.

Les vitamines A et E sont préparées depuis longtemps par voie chimique. Les procédés de préparation sont nombreux et variés, on trouve un exposé relativement exhaustif des procédés utilisés industriellement dans un article publié par J. Paust dans Pure & Appl. Chem., Vol. 63, No 1, pp. 45-58, 1991.

Dans cet article, Paust décrit différentes techniques industrielles pour effectuer la condensation des différents motifs polyéniques pour accéder à la vitamine A. Cette condensation est réalisée soit par l'intermédiaire d'une sulfone (brevet DE 2.202.689), soit par l'intermédiaire d'un dérivé acétylénique engagé dans une réaction de Grignard (article paru dans Pure Appl. Chem. 47,183 (1976) ou enfin par une réaction de Wittig mettant en jeu un ylure de phosphore (brevets DE 1.046.046 et DE 1.026.745).

Chacune de ces trois techniques de condensation de différents motifs polyéniques utilise comme matière de départ un intermédiaire relativement onéreux qui seul, dans chacun des procédés décrits, va permettre de réaliser la condensation, cela rend cette étape dans le cadre de tous les procédés décrits particulièrement onéreuse.

En plus du fait que les intermédiaires de départ sont onéreux, dans tous ces procédés, une étape de recyclage ou d'élimination des produits secondaires formés est nécessaire. Le phosphonium se transforme en oxyde de phosphine qu'il faut réduire en phosphine, le dérivé sulfonique produit en fin de réaction un phénylsulfinate qu'il faut recycler, le dérivé acétylénique consomme, lors de la réaction, du magnésium générateur de sels qu'il faut éliminer.

Nous avons donc cherché un nouveau procédé de préparation des vitamines A et E, permettant de s'affranchir de ces composés.

Notre invention concerne donc un nouveau procédé de condensation de motifs polyéniques n'utilisant ni les dérivés sulfoniques, ni les sels de phosphonium, ni les motifs acétyléniques. Elle concerne plus particulièrement un procédé qui consiste à condenser un dérivé monoinsaturé ou polyinsaturé éventuellement porteur d'un hétéroatome, de préférence d'un atome de soufre, de sélénium, ou d'un halogène, comportant 2 à 20 atomes de carbone, la condensation étant réalisée sur un dérivé d'un alcool allylique éventuellement polyinsaturé ou sur un dérivé porteur d'une fonction alcoxyle ou d'une fonction silyloxy insaturé, également éventuellement polyinsaturé, comportant lui 3 à 20 atomes de carbone.

La réaction de condensation est effectuée en présence d'un acide de Lewis ou d'un acide protique.

Cette réaction permet, entre autres, la formation d'intermédiaires nouveaux de la vitamine A qui répondent à la formule (I) suivante : 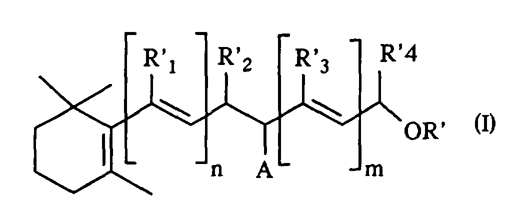 dans laquelle:
- R'₁, R₂'_{,} R'₃ et R'₄ représentent chacun un motif semblable ou différent, dans chacun des motifs insaturés, choisi parmi les groupes hydrogène ou alkyle,
- A représente un atome d'halogène choisi parmi le chlore ou le brome, un groupe arylthio, ou arylséléno,
- R' représente un atome d'hydrogène, une liaison avec l'atome de carbone de la chaîne, un groupe alkyle ou un groupe alkylcarbonyle,
- n et m sont des nombres entiers égaux à 0, 1, 2 ou 3, A n'étant pas un brome ou un chlore lorsque m est égal à 2 et n est égal à 1 et lorsque m est égal à 3 et n est égal à 0.

On préfère parmi l'ensemble des composés de formule (I) ceux pour lesquels A représente le chlore et m+n est égal à 3.

Il est connu d'après le brevet de Oediger, Kabbe et Eiter publié sous le numéro DE 1.157.606 de préparer le dérivé de formule (I) dans lequel A représente un atome de brome ou de chlore, n est égal à 1, m est égal à 2 et R' représente un motif acétate. Ce dérivé est préparé à partir de l'alcool correspondant en C20 par exemple par action du tribromure de phosphore dans le cas du dérivé bromé, le dit alcool est lui même préparé par action de l'aldéhyde en C14 avec un dérivé acétylénique en C6 selon la réaction suivante suivie d'une hydrogénation partielle de la liaison acétylénique :

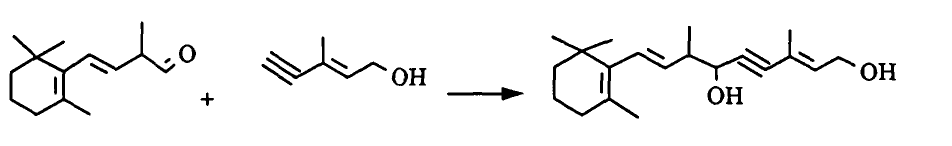

Il est impossible par cette technique d'introduire le groupe hydroxyle et, par la suite, un atome d'halogène à une autre place qu'en position 10 sur la chaîne de la vitamine A, la présence du motif acétylénique imposant cette place.

Aussi parmi les composés de formule (I) on préfère les composés pour lesquels A représente le chlore et pour lesquels n+m est égal à trois car ils permettent l'accès direct à la vitamine A après déshydrohalogénation.

Le composé de formule (I) dans lequel A représente un halogène, n est égal à 0, m est égal à 3 et R' est H, est également connu (US-P-2,451,739 entre autres).

La présente invention concerne également des intermédiaires de la vitamine E ou des caroténoïdes qui répondent à la formule suivante : 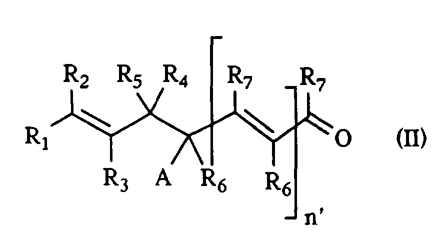 dans laquelle:
- R₁, R₂, R₃, R₄, R₅, R₆ et R₇ représentent chacun un groupe semblable ou différent choisi parmi les motifs hydrogène, alkyle, alkényle éventuellement cyclique, deux des substituants pouvant former entre eux un cycle insaturé ou saturé,
- A représente un groupe halogène choisi parmi le chlore et le brome, un groupe arylthio ou arylséléno,
- n' est un nombre entier compris entre 0 et 10.

Parmi l'ensemble des formules (I) et (II), on préfère celles pour lesquelles A représente le chlore.

L'objet de notre invention concerne également la préparation des composés de formule (I) et (II).

Ce procédé de préparation consiste comme indiqué précédemment à mettre en présence un dérivé mono ou polyinsaturé éventuellement porteur d'un atome d'halogène, d'un groupe arylthio, arylséléno, acyloxy, trialkylsilyle avec un dérivé d'un alcool allylique, éventuellement polyinsaturé, ou un dérivé porteur d'une fonction alcoxy ou silyloxy mono ou polyinsaturé.

Les dérivés mono ou polyinsaturés éventuellement porteurs d'un hétéroatome choisi parmi les atomes d'halogène, les groupes arylthio, arylséléno, acyloxy, trialkylsilyle répondent à la formule (III) suivante: 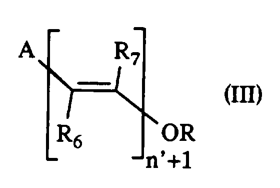 dans laquelle :
- R représente un groupe alkyle ou silyle ayant un à quatre substituants carboné, un groupe alkylcarbonyle ou tosyle,
- R₇ et R₆ représentent chacun, dans chacun des motifs insaturés, un substituant identique ou différent, choisi parmi l'hydrogène ou parmi les radicaux alkyle ayant un à quatre atomes de carbone,
- A représente l'hydrogène, un atome d'halogène choisi parmi le chlore ou le brome, un goupe arylthio, arylséléno, acyloxy, trialkylsilyle,
- n' représente un nombre entier compris entre 0 et 10.

Les dérivés d'un alcool allylique ou porteurs d'une fonction alcoxy ou silyloxy, insaturés, éventuellement polyinsaturés répondent à la formule (IV) suivante: 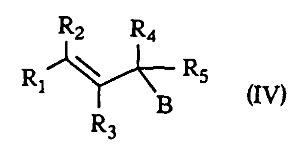 dans laquelle:
- R₁, R₂, R₃, R4 et R₅ représentent l'hydrogène, un motif alkyle ayant un à vingt atomes de carbone, un motif alkényle ayant deux à vingt atomes ou peuvent former entre eux une chaîne terpénique polyénique alkylène ou alkénylène cyclique, éventuellement substituée,
- B représente un groupe hydroxyle,alcoxyle ayant 1 à 6 atomes de carbone, alcoyle, aryloxy ou silyloxy ou halogène.

Pour la préparation de la vitamine A plus spécifiquement on met en oeuvre de préférence les dérivés de formule (IV) de formules suivantes :
- pour les dérivés ayant 10 atomes de carbone : 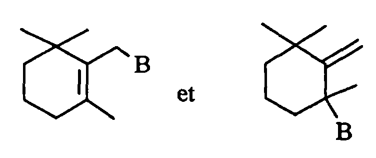
- pour les dérivés ayant 13 atomes de carbone : 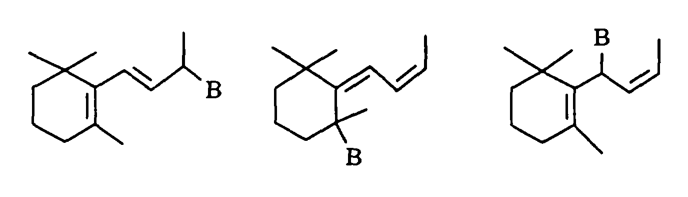
- pour les dérivés ayant 15 atomes de carbone : 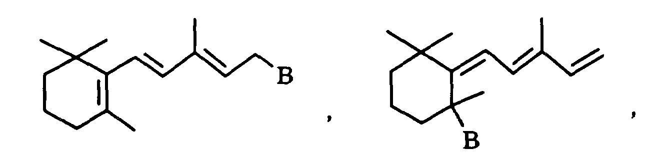 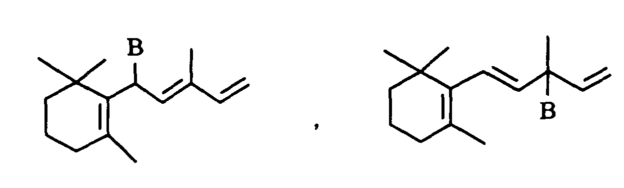
- pour les dérivés ayant 18 atomes de carbone :

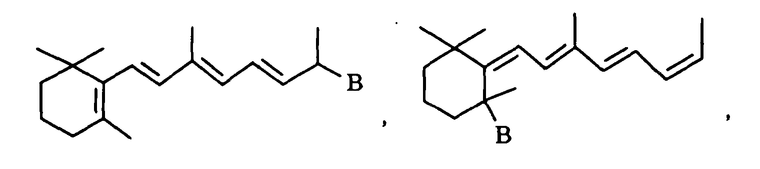

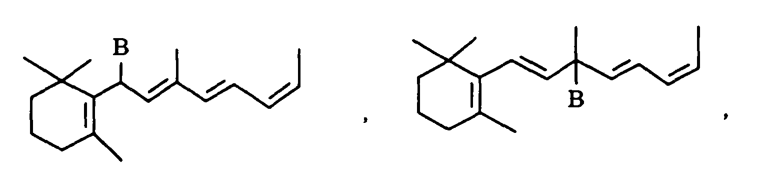

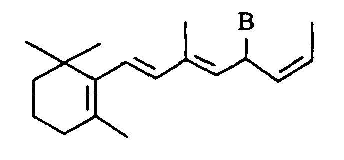

Toujours en ce qui concerne plus spécifiquement la synthèse de la vitamine A, parmi les dérivés mono ou polyinsaturés de formule (III), on préfère utiliser les composés suivants :
- pour les dérivés ayant 10 atomes de carbone : 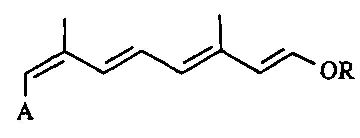
- pour les dérivés ayant 7 atomes de carbone : 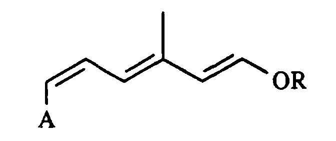
- pour les dérivés ayant 5 atomes de carbone : 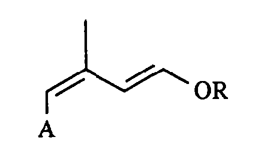
- pour les dérivés ayant trois atomes de carbone : 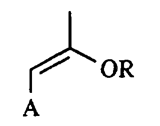
- et pour les dérivés ayant 2 atomes de carbone :

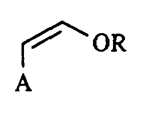

Le procédé de préparation des composés de formule (III) consiste à effectuer un craquage thermique des acétals de formule (V) suivante : 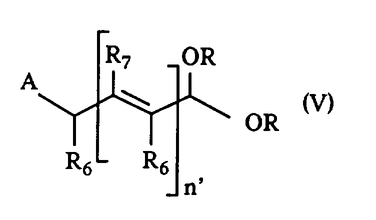 dans laquelle A, R₇, R₆, R et n' ont la même signification que précédemment.

Le craquage peut être réalisé en présence d'une quantité catalytique d'un acide choisi parmi: les acides p. toluène sulfonique, toluique, le chlorure de zinc, les catalyseurs hétérogènes acides ...

La réaction de condensation du dérivé de formule (III) avec le dérivé de formule (IV) est réalisée en présence d'un acide de Lewis ou d'un acide protique. Ces acides sont choisis parmi notamment: le chlorure de zinc, l'éthérate de trifluorure de bore, le chlorure ferrique, le triflate de triméthylsilyle, les chlorures stanneux et stanniques, l'acide formique, l'acide trichloroacétique, l'acide trifluoroacétique, l'acide trifluorométhanesulfonique, le triflate de diméthyl tertiobutylsilyle, les catalyseurs hétérogènes acides (résines Nafion), le trichlorure de bismuth, le tétrachlorure de titane.

Le solvant de réaction est choisi parmi les solvants tels que notamment les alcools, l'eau, les nitriles, les nitroalkanes, les nitroaryles, les solvants aliphatiques ou aromatiques halogénés, les sulfones ou les acides organiques, il est bien entendu que la réaction peut aussi être réalisée dans un mélange de ces solvants.

La condensation peut être réalisée, selon une meilleure manière de mettre en oeuvre l'invention, en présence d'un alcool et/ou d'eau.

La température réactionnelle est comprise de préférence entre -45°C et 50°C et encore plus préférentiellement entre -45°C et la température ambiante.

La dernière étape du procédé selon l'invention consiste, lorsque A représente un atome d'halogène, à déhydrohalogéner la molécule de formule (I) ou (II) obtenue. Cette étape est réalisée conformément à l'enseignement de la publication de Oediger, Kabbe, Möller et Eiter parue dans Chem. Ber. 99(6), 2012-2016(1966).

L'agent de déhydrohalogénation est choisi notamment parmi :
- le chlorure de lithium dans le diméthylformamide,
- le diazabicyclooctane (DABCO),
- le diazabicyclononène (DBN),
- le diazabicycloundécène (DBU),
- la quinuclidine,
- la potasse ou la soude dans un hydrocarbure en présence éventuellement d'un agent de transfert de phase,
- le palladium zéro en présence d'une base.

Lorsque A représente un groupe sulfinyle on utilise avantageusement une étape de mise en solution dans le tétrachlorure de carbone.

La présente invention sera plus complètement décrite en présence des exemples suivants qui ne doivent pas être considérés comme limitatifs de l'invention.

### EXEMPLE 1 Préparation du composé de Formule III

### 1) Craquage de l'acétal bromé

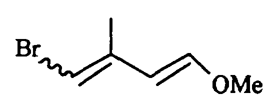

Dans un appareil à distillation, on introduit 8 grammes de 1-bromo-2-méthyl4,4-diméthoxy but-2-ène (38,28 mmol). On chauffe le produit, sous agitation, à pression atmosphérique, jusqu'à l'apparition de vapeurs sur le surnageant. On arrête alors de chauffer et porte sous le vide d'une trompe d'eau. On distille ainsi un mélange de l'acétal de départ et de l'éther d'énol attendu. Ce dernier est purifié par une chromatographie éclair sur silice, et on en obtient 1.7 g. Rdt = 25 %.

### 2) Craquage de l'acétal chloré

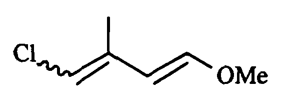

Dans un appareil à distillation, on introduit 10 grammes de 1-chloro-2-méthyl-4,4 diméthoxy but-2-ène (60,8 mmol). On chauffe le produit jusqu'à distillation complète du méthanol. Puis on distille 7,6 g d'éther d'énol chloré sous le vide de la trompe à eau (rendement 94%).

### 3) Formation et craquage du phénylthioacétal

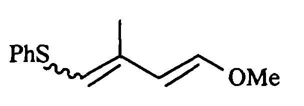

On introduit 20 grammes de 1-chloro-2-méthyl-4,4 diméthoxy but-2-ène (0,122 mol) dans 68 ml de triéthylamine, et on ajoute, au goutte à goutte à l'aide d'une ampoule à brome en 10 mn à température ambiante, 14 g de thiophénol (soit 1 eq) en solution dans 65 ml d'éther. On laisse sous agitation à cette température pendant 15 heures, filtre, lave le chlorhydrate de triéthylamine par 30 ml d'éther. Les phases éthérées sojt concentrées puis le résidu est placé dans un appareil à distiller. On le chauffe jusqu'à distillation complète du méthanol, puis on distille 20 g de méthoxy-1 méthyl-3 phénylthio-4 butadiène-1,3. Le rendement par rapport au chloro-acétal de départ est de 80 %.
Eb (0,6 mmHg) = 129°C.

### 4) Formation et craquage du phénylsélénoacétal

On met en oeuvre la réaction suivante :

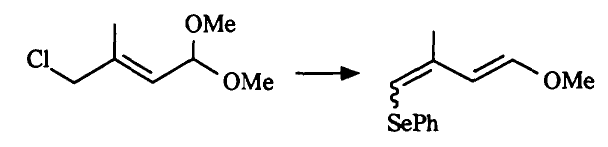

Sur une solution de 12 grammes (38.5 mmol) de diphényldiséléniure dans 200 ml d'éthanol, on ajoute à 0°C, et sous argon, 3.1 g (2.1 eq.) de borohydrure de sodium par petites fractions la réaction est exothermique.

On laisse sous agitation le temps de voir cesser le dégagement d'hydrogène, puis on ajoute au goutte-à-goutte 12.7 g (2 eq.) de 1-chloro-2-méthyl-4,4 diméthoxy but-2-ène. Après 15 heures sous agitation à température ambiante, on filtre, concentre, cristallise le résidu de NaCl dans l'éther de pétrole, puis introduit le produit brut dans un montage à distillation. Le point de craquage commence vers 120°C, et on distille à 150°C sous 0.6 mmHg 12.7 g de phénylsélénoéther d'énol attendu : le rendement est de 65 %.

### 5) Formation et craquage du phénylthioacétal en C₂

On met en oeuvre la réaction suivante :

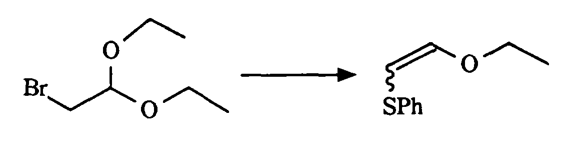

Dans 200 ml d'éthanol absolu, on ajoute par petites fractions 5.75 g de sodium métallique (0.25 mol), et attend la disparition complète de celui-ci. On ajoute au goutte-à-goutte 27.5 g (0.25 mol) de thiophénol, puis 38 ml de 1-bromo-2,2-diéthoxyéthane (1 eq), on chauffe au reflux pendant 2 heures, verse la solution dans 500 ml d'eau, puis extrait au chloroforme, sèche sur MgSO₄ : le produit brut est introduit dans un montage à distillation. Le craquage commence vers 120°C, et on distille ensuite l'éther d'énol attendu. Le rendement est de 30 %.

### 6) Formation et craquage de l'acétoxyacétal en C₅

### 1-acetoxy-4,4-diméthoxy-2-méthyl-but-2-ene

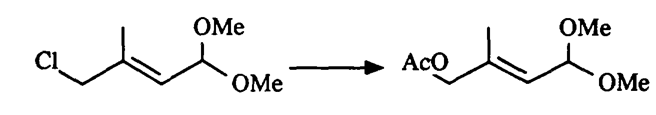

On porte au reflux pendant 24 heures une solution de 30 g de chloracétal (0.182 mol) en C₅ et 18 g d'acétate de potassium (1 eq) dans 75 ml de méthanol. On laisse ensuite revenir à température ambiante, puis concentre, reprend par 100 ml d'éther, lave par 2 fois 30 ml d'eau, sèche sur sulfate de magnésium, filtre et concentre. Le rendement est de 65 %.

### 1-acétoxy-4-méthoxy-2-méthyl-buta-1,3-diene

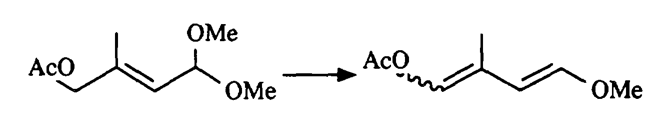

L'acétal brut est placé dans un appareil de Claisen, puis chauffé jusqu'à au moins 120°C, température à laquelle le craquage semble avoir lieu. On distille ensuite l'éther d'énol attendu. Le rendement est de 60 % (Masse obtenue : 17 g) Eb_{0.4mmHg}= 75-82°C. 2 isomères dans les proportions 50/50.

### EXEMPLE 2 Préparation du composé de formule I (A=SPh)

### Réaction du phénylthio-éther d'énol sur le vinyl béta-ionol, en présence d'éthérate de trifluorure de bore.

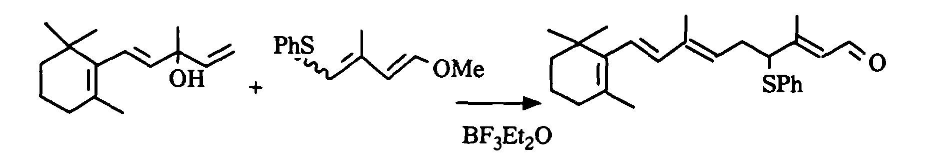

Dans un bicol préalablement purgé à l'argon, on introduit 0,61 g de phénylthio-éther d'énol (2,96 mmol) dans 3 ml de nitroéthane. On porte à -35°C, puis ajoute, au pousse seringue en 2 mn, à cette température, une solution de 0,66 g de vinyl béta-ionol (1 eq) dans 3 ml de nitroéthane, on laisse sous agitation pendant 10 mn, puis on ajoute rapidement 75 µL d'éthérate de trifluorure de bore (0,2 eq). La solution devient verte. On laisse sous agitation entre -30 et -35°C pendant une heure, puis on ajoute rapidement 4,5 ml d'une solution aqueuse saturée en hydrogénocarbonate de sodium. On laisse remonter à température ambiante, puis on agite pendant 15 mn. On ajoute 5 ml de dichlorométhane, puis on lave la phase organique par 2 fois 5 ml d'eau. La phase organique est séchée sur sulfate de magnésium, filtrée, concentrée, et le produit brut est chromatographié sur silice. On recueille ainsi 580 mg de phénylthio-rétinal, soit un rendement de 50 %.

### Accès à l'acétate de vitamine A

### Réduction de l'aldéhyde en alcool par le borohydrure de sodium

On met en oeuvre la réaction suivante :

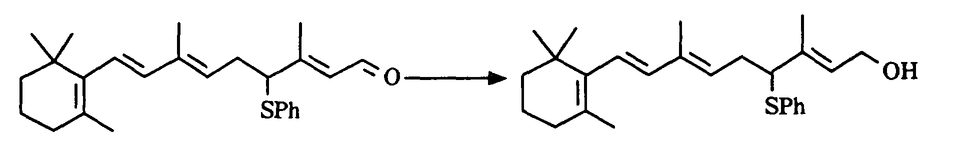

Sur une solution de 0.57 g d'aldéhyde précédent en C20 (1.45 mmol) dans 6 ml de méthanol, on ajoute à 0°C 55 mg de borohydrure de sodium (1.45 mmol), puis laisse revenir à température ambiante en 15 mn. On laisse la nuit sous agitation (environ 15 heures), puis ajoute 15 ml d'eau, extrait par 2 fois 10 ml d'éther, lave par 10 ml d'eau, sèche sur sulfate de magnésium, filtre, concentre pour recueillir 0.57 g d'alcool, soit un rendement de 99.5 %.

### Acylation de l'alcool en acétate

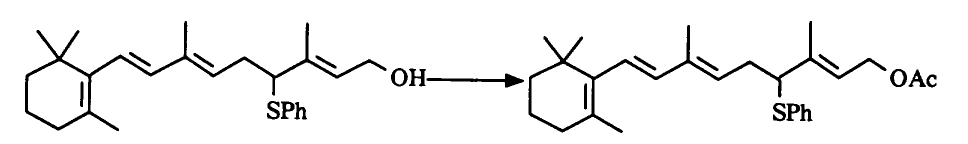

Sur une solution de 0.37 g d'alcool en C20 (0.93 mmol) dans 9 ml d'hexane, on introduit successivement 0.21 ml de triéthylamine (1.5 mmol ; 1.6 eq) et 0.17 ml d'anhydride acétique (1.8 mmol ; 1.9 eq). Après 7 heures d'agitation à 40°C, on constate la disparition totale du spot de l'alcool initial en CCM. On ajoute alors 7 ml d'eau glacée, puis reprend par 5 ml d'éther, et lave par 5 ml d'eau. On sèche sur sulfate de magnésium, filtre et concentre pour recueillir 405 mg d'acétate en C20. Le rendement est quantitatif.

### Oxydation de l'acétate

On met en oeuvre la réaction suivante :

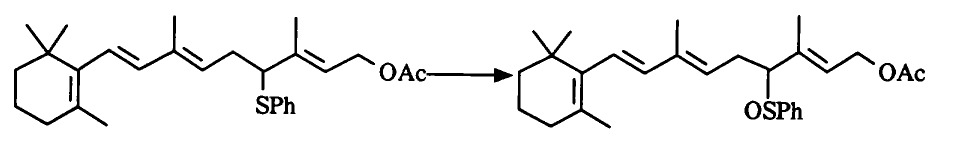

Sur une solution de 0,455 g (1.04 mmol) d'acétate précédent dans 10 ml de dichlorométhane refroidi à 0°C, on verse 0,206 g (1.15 eq) d'acide métachloroperbenzoïque dans 2.5 ml de dichlorométhane.

La réaction est totale en 1 heure à 0°C. On hydrolyse par addition rapide de 5 ml d'une solution aqueuse saturée en hydrogénocarbonate de sodium, sépare les deux phases, lave la phase organique par 2 fois 15 ml de soude normale, sèche sur sulfate de magnésium, filtre et concentre. Le rendement brut est de 100 %.

### Déhydrosulfoxydation sur l'acétate : accès à l'acétate de vitamine A

On met en oeuvre la réaction suivante :

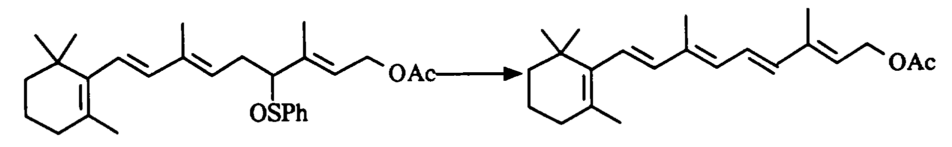

On place 0.51 g de phénylsulfoxyde précédent en C20 (1.12 mmole) dans 4 ml de tétrachlorure de carbone, et porte au reflux la solution : il faut 3 h 30 mn pour voir disparaître le sulfoxyde en CCM : on concentre puis chromatographie le résidu sur alumine Activité II (Eluant : Ether de Pétrole/Et₂0 = 97/3). On recueille ainsi 0,28 g d'acétate de vitamine A. Le rendement est de 76 %.

### Accès au Rétinal

### Oxydation de l'aldéhyde

On met en oeuvre la réaction suivante :

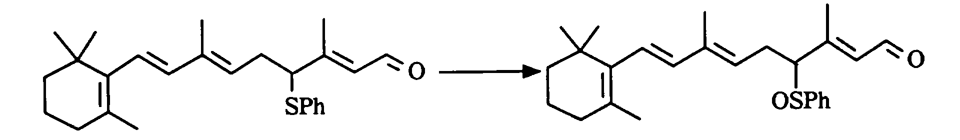

Sur une solution de 0,41 g (1.04 mmol) d'aldéhyde obtenu à l'exemple 2 dans 10 ml de dichlorométhane refroidi à 0°C, on verse 1.15 eq. d'acide méta-chloroperbenzoïque dans 2.5 ml de dichlorométhane.

La réaction est totale en 1 heure à 0°C. On additionne rapidement 5 ml d'une solution aqueuse saturée en hydrogénocarbonate de sodium, sépare les deux phases, lave la phase organique par 2 fois 15 ml de soude normale, sèche sur sulfate de magnésium, filtre et concentre. Le rendement brut est de 100 %.

### Déhydrosulfoxydation sur l'aldéhyde : accès au rétinal

On met en oeuvre la réaction suivante :

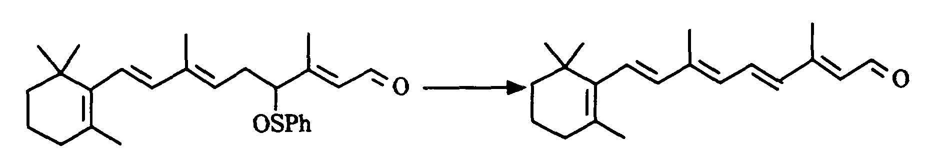

On place 0,43 g de phénylsulfoxyaldéhyde en C20 (1.05 mmol) dans 8 ml de tétrachlorure de carbone et on suit la disparition du sulfoxyde par CCM. La réaction semble instantanée à température ambiante. Après 1 heure, on concentre et chromatographie le résidu sur silice (Eluant : Ether de Pétrole/Et₂O = 95/5). On recueille ainsi 0,275 g de rétinal. Le rendement est de 92 %.

### EXEMPLE 3 Préparation du composé de formule (I) (A=CI)

### Synthèse et application d'un dérivé du rétinal

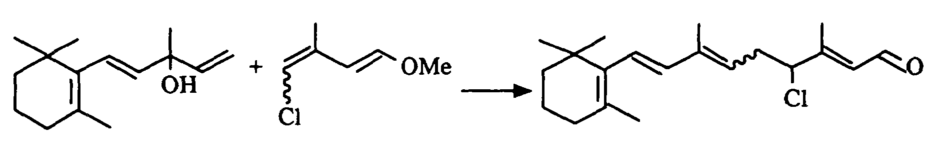

### 1) Conditions standards

On mélange à température ambiante 0,41 g d'éther d'énol chloré et 0,66 g de vinyl-β-ionol (soit 3 mmol de chaque) dans 6 ml de nitroéthane. La solution est portée à -35°C dans un bain thermostaté et on ajoute rapidement le catalyseur (0,5 eq de chlorure de zinc ou 0,2 eq d'éthérate de trifluorure de bore). Après 10 mn sous agitation à -35°C, on ajoute rapidement 5 ml d'une solution aqueuse saturée en hydrogénocarbonate de sodium, puis on laisse revenir à température ambiante en 10 mn. On extrait par 2 fois 5 ml de dichlorométhane, puis on lave par 5 ml d'eau. On sèche sur sulfate de magnésium, on filtre et on concentre. On recueille, après chromatographie éclair sur silice (éther de pétrole/éther éthylique : 96/4) 0,400 g d'aldéhyde chloré en C20, soit un rendement de 42 %. Le composé obtenu est analysé par RMN et infrarouge.

### Conditions communes pour les tableaux 1-1 à 1-8

Les 2 réactifs sont introduits dans le solvant en quantités équimolaires (sauf indication contraire) à la concentration de 0,5 M. Le milieu réactionnel est porté à la température indiquée, puis on ajoute le catalyseur et laisse réagir pendant le temps indiqué.

### 1-1) Influence du catalyseur

### Conditions communes : 1 eq d'isopropanol, 10 mn à -35°C, dans le nitroéthane

| CATALYSEUR | QUANTITE | RENDEMENT |
|---|---|---|
| CF₃SO₃H | 0.2 eq | 47% |
| CF₃SO ₃SiMe₃ | 0.2 eq | 46 % |
| CF₃SO₃SiMe₂tBu | 0.2 eq | 36 % |
| SbCl₃ | 0.2 eq | 34 % |
| SnCl₄ | 0.2 eq | 30 % |
| CF₃COOH | 0.2 eq | 25 % |
| HCOOH | solvant | 22 % |
| SnCl₂ | 0.2 eq | 20 % |
| BiCl₃ | 0.2 eq | 16 % |

### 1-2) Influence de la température

### Conditions communes : une heure dans le nitroéthane.

| CATALYSEUR | TEMPERATURE EN °C | RENDEMENT |
|---|---|---|
| 0,2 eq BF₃-Et₂O | -45 | 31 % |
| Idem | - 35 | 32 % |
| Idem | -20 | 25 % |
| Idem | 0 | 25 % |
| 0,2 eq ZnCl₂ | 0 | 27 % |
| Idem | - 35 | 35 % |

### 1-3) Influence du temps

### Conditions communes : Température de -35°C, solvant nitroéthane.

| CATALYSEUR | TEMPS EN MIN | RENDEMENT |
|---|---|---|
| 0,5 eq ZnCl₂ | 5 | 40 % |
| Idem | 10 | 42 % |
| Idem | 15 | 28 % |
| Idem | 60 | 12 % |
| 0,2 eq BF₃ Et₂O | 60 | 32 % |
| Idem | 10 | 41 % |

Ce tableau montre, d'une manière générale, que la réaction est très rapide.

### 1-4) Nature du solvant et concentration

### Conditions communes : 10 mn à -35°C; 0,5 eq de chlorure de zinc.

| SOLVANT | CONCENTRATION | RENDEMENT |
|---|---|---|
| EtNO₂ | 0,5 M | 42 % |
| EtNO₂ | 1 M | 35 % |
| CH₂Cl₂ | 0,5 M | 21 % |

### 1-5) Influence de l'ajout d'alcool

### Conditions communes : 0,5 eq de chlorure de zinc, 10 mn à -35°C dans le dichlorométhane.

| ALCOOL | RENDEMENT |
|---|---|
| Sans | 42 % |
| isopropanol | 53 % |
| terbutanol | 51 % |
| 2-pentanol | 43 % |
| isobutanol | 38 % |

### 1-6) Influence de l'ajout d'un acide protique

### Conditions communes: mélange acide carboxylique/eau 5/1 vol/vol utilisé comme solvant

| CATALYSEUR | TEMP.°C | DUREE | RENDEMENT |
|---|---|---|---|
| HCOOH | -15°C | 15' | 60 % |
| CCl₃COOH | -15°C | 3' | 30 % |
| CF₃COOH | -25°C | 0,5' | 18 % |

### 1-7) Agent d'hydrolyse

Après condensation dans les mêmes conditions : 10 mn à -35°C dans le nitroéthane ; 0,5 eq de chlorure de zinc, on utilise deux agents d'hydrolyse différents.

| AGENT D'HYDROLYSE | RENDEMENT |
|---|---|
| NaHCO₃ sat/H₂O (excès) | 42 % |
| Triéthylamine (0,5 eq) | 40 % |

### 1-8) Proportions respectives des réactifs

### 10 minutes à -35°C dans le nitroéthane; 0,5 eq de chlorure de zinc.

| ETHER D'ENOL | VINYL β IONOL | RENDEMENT |
|---|---|---|
| 1 | 1 | 42 % |
| 2 | 1 | 42 % |
| 1 | 2 | 15 % |

### 1-9) Stéréochimie

L'analyse RMN à haut champ montre que l'aldéhyde chloré en C20 est un mélange des stéréoisomères suivant : 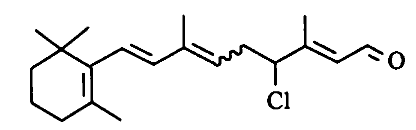
Tout trans : 65 %
7 trans, 9 cis, 13 trans : 35 %

### Application à la préparation de la vitamine A

### 1) Déhydrochloration de l'aldéhyde au chlorure de lithium

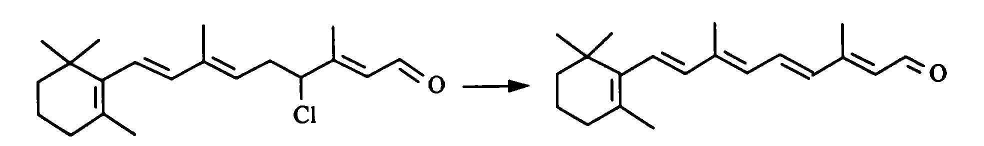

On porte à 100°C un mélange d'aldéhyde chloré en C20 125 mg (0,39 mmol) et 60 mg de chlorure de lithium sec (soit 3 eq) dans 6 ml de diméthylformamide anhydre. Après 10 mn à 100°C, on constate la disparition du produit de départ en chromatographie couche mince. On laisse revenir la solution à température ambiante en 10 mn, puis ajoute 50 ml d'éther et lave par 2 fois 20 ml d'eau et 2 fois 20 ml d'une solution aqueuse saturée en hydrogénocarbonate de sodium. On sèche sur sulfate de magnésium, filtre et concentre. Le brut est chromatographié sur silice (éther de pétrole/éther éthylique : 98/2) et on recueille 83 mg de rétinal, soit un rendement de 75 %.

Analyse HPLC : λ = 330 nm ; débit = 1,5 ml/mn ; P = 70 bars ; éluant : hexane/éther éthylique= 9/1
Tout trans : 44,9 %
9 Cis : 16,8 %
13 Cis : 38,3 %.

### 2) Déhydrochloration de l'aldéhyde au diazabicycloundécène (DBU)

A une solution de 270 mg d'aldéhyde chloré en C20 dans 1,5 ml de dichlorométhane, on ajoute rapidement à température ambiante 0,13 ml de DBU (soit 1,05 eq), et on laisse sous agitation à cette température pendant 24 heures. On évapore le dichlorométhane, on reprend par 10 ml d'éther, on lave par 2 fois 5 ml d'une solution aqueuse saturée en hydrogénocarbonate de sodium puis 5 ml d'eau, puis on sèche sur sulfate de magnésium, filtre, concentre. Le brut est chromatographié sur silice (éther de pétrole/éther éthylique : 98/2), et on recueille 203 mg de rétinal. Rendement 85 %.

Analyse HPLC : λ = 330 nm ; débit = 1,5 ml/nm ; P = 70 bars ; éluant : hexane/éther = 9/1
Tout trans: 58,7 %
9 Cis : 7,7 %
13 Cis : 33,6 %.

### 3) Déhydrochloration par un mélange de PdCl₂,P(C₆H₅)₃

A une solution d'aldéhyde chloré en C20 (99mg; 0,31 mmol), et de triéthylamine (47 mg; 0,46 mmol) dans 2 ml de diméthylformamide, on ajoute sous argon à 60°C, PdCl₂ (5,4 mg; 0,032 mmol) et P(C₆H₅)₃ (20,3 mg; 0,77 mmol). On laisse sous agitation à 60°C pendant 2 heures. On extrait de la même façon qu'à l'exemple 2. On obtient le rétinal avec un rendement de 78 %.

### 4) Réduction de l'aldéhyde en alcool

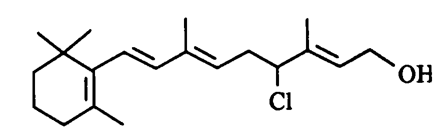

A une solution de 250 mg (0,78 mmol)d'aldéhyde chloré en C20 dans 10 ml de THF, on ajoute, à 0°C en 1 mn, 30 mg de borohydrure de sodium, puis laisse sous agitation à 0°C pendant 5 heures. On ajoute alors 5 ml d'eau puis on extrait par 2 fois 10 ml d'éther, on lave par 10 ml d'eau, sèche sur sulfate de magnésium, filtre et concentre : on recueille 250 mg d'alcool chloré, soit un rendement de 99 %.
IR (Film, cm⁻¹) : 3400, 2940, 2905, 2850 ; 1445 ; 1370.

### 5) Acylation de l'alcool en acétate

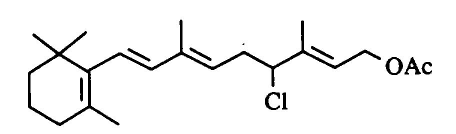

A une solution de 250 mg d'alcool chloré, obtenu ci-dessus, dans 9 ml d'hexane on ajoute successivement 0,21 ml de triéthylamine et 0,17 ml d'anhydride acétique. On laisse réagir 15 heures à température ambiante puis 2 heures à 40°C pour obtenir 268 mg d'acétate chloré en C20, soit un rendement de 95 %.

### 6) Déhydrochloration de l'acétate au diazabicyclononène (DBN)

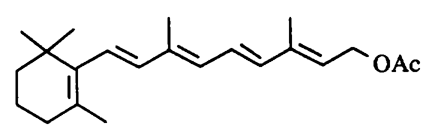

A une solution d'acétate chloré en C20 1,5 g (4,12 mmoles) dans 3 ml de toluène, on ajoute rapidement une solution de 1 g de diazabicyclononène (DBN) (soit 2 eq) dans 3 ml de toluène. On porte à 80°C pendant 15 mn puis on laisse refroidir et on verse sur 10 g de glace contenant 1 ml d'une solution aqueuse molaire en acide sulfurique. On extrait par 3 fois 10 ml d'éther de pétrole, lave par 10 ml d'une solution aqueuse saturée en hydrogénocarbonate de sodium, sèche sur sulfate de magnésium, filtre et concentre pour obtenir 1,29 g d'acétate de vitamine A, soit un rendement de 95,5 %.

Analyse HPLC : λ = 280 nm ; débit = 1,5 ml/nm ; P = 70 bars ; éluant : hexane/éther = 97/3
Tout trans : 79,6 %
9 Cis : 12%
13 Cis : 8,4 %.

### EXEMPLE 4 Obtention d'un dérivé de formule I (A=SePh) par action d'un phénylséléno-éther d'énol en C₅ sur le vinyl-β-ionol :

On met en oeuvre la réaction suivante :

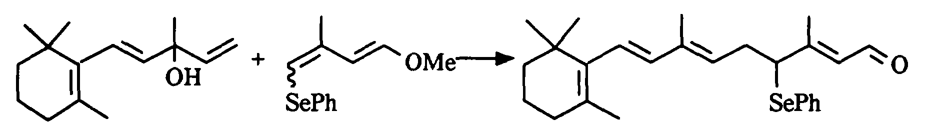

A un mélange de 0,660 g (3 mmol) de vinyl-β-ionol et 0,855 g (1 eq.) d'éther d'énol préalablement porté à 0°C, on ajoute 5 ml d'acide formique et 1 ml d'eau, préalablement refroidis à 0°C. On laisse réagir pendant 60 mn à 0°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4) : le rendement est de 48 %.

### Accès au Rétinal

On met en oeuvre la réaction suivante :

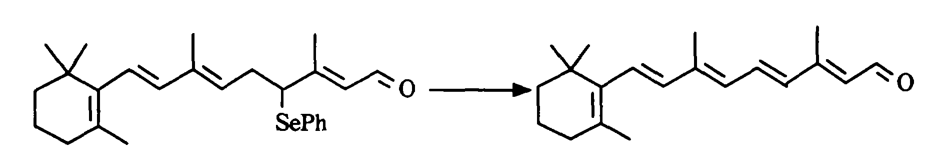

Sur une solution de 0,500 g (1.13 mmol) de dérivé I (A = SePh), en solution dans 10 ml de dichlorométhane, on ajoute, à 0°C, 210 mg (1.1 eq.) d'acide métachloroperbenzoïque en solution dans 5 ml de dichlorométhane : la formation du rétinal est immédiate. On traite aussitôt par 5 ml d'une solution aqueuse d'hydrogénocarbonate de sodium, extrait par 5 ml de dichlorométhane, lave par 2 fois 3 ml de soude 0.5 N, sèche sur sulfate de magnésium, filtre et concentre. Le rendement est de 90 % en rétinal purifié par chromatographie éclair sur silice (Eluant : Eth. Pet./Et₂O = 96/4).

### Exemple 5 Obtention d'un dérivé de formule I (A=Br) par action d'un bromo-éther d'énol en C₅ sur le vinyl-β-ionol

On met en oeuvre la réaction suivante :

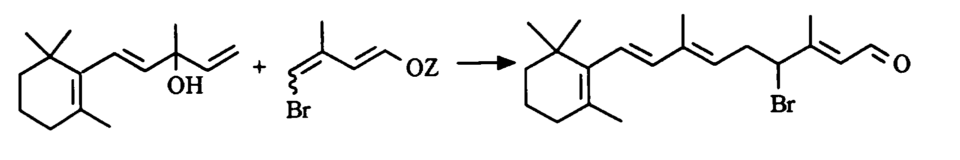

Z = Et ou SiMe₃

A un mélange de 0,660 g (3 mmol) de vinyl-β-ionol et (3 mmol) (1 eq.) d'éther d'énol préalablement porté à -10°C, on ajoute 5 ml d'acide formique et 1 ml d'eau, préalablement refroidis à -10°C. On laisse réagir pendant 60 mn à -10°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant ; Eth. Pet./Et₂O = 94/4). Le rendement est de 32 % pour Z = Et, et 28 % pour Z = SiMe₃.

### Accès au Rétinal

On met en oeuvre la réaction suivante :

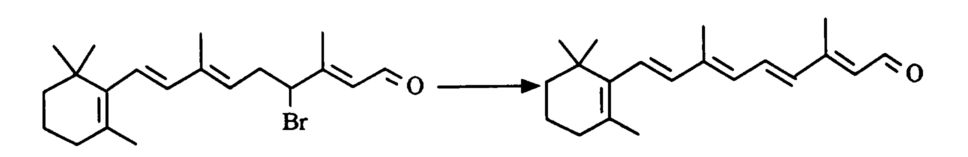

On place 0.22 g de rétinal hydrobromé (0.6 mmol) dans 3 ml de dichlorométhane, puis ajoute 92 microlitres (1 eq.) de DBU. On laisse sous agitation pendant 1 heure puis concentre et reprend par 5 ml d'éther, lave par 2 fois 3 ml d'eau, sèche sur sulfate de magnésium, filtre et concentre pour obtenir 0,165 g de rétinal.
Rendement = 96.5 %.

### Exemple 6 Obtention d'un dérivé de formule I (A=Br) par action d'un éther d'énol en C₂ sur un dérivé en C₁₈

On met en oeuvre la réaction suivante :

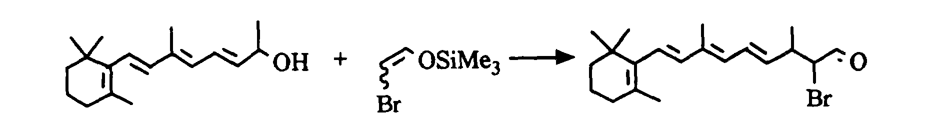

On place 933 mg d'alcool en C18 (3.6 mmol) et 3.6 mmol (1 eq.) de 1-bromo 2-triméthylsiloxy éthylène dans 7.2 ml de nitroéthane, porte à -35°C, et ajoute 90 microlitres d'éthérate de trifluorure de bore. On laisse sous agitation pendant 1 heure à -35°C, puis hydrolyse par 6 ml d'une solution aqueuse d'hydrogénocarbonate de sodium, extrait au dichlorométhane.

Le produit attendu est caractérisé en RMN¹H.

RMN¹H(CDCl₃) : 9.35 (d,1H,J = 8.75 Hz).

L'alcool en C18 est obtenu par action de l'aluminohydrure de lithium sur la cétone C18 dans l'éther anhydre à 0°C.

### Exemple 7 Procédé de préparation du rétinal par action d'un acyloxyéther d'énol en C₅ sur le vinyl-β-ionol

On met en oeuvre la réaction suivante :

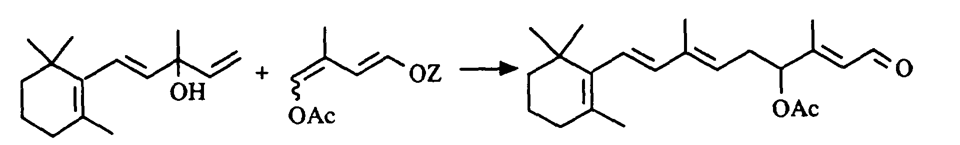

Z = Me ou SiMe₃

A un mélange de 660 mg (3mmol) de vinyl-β-ionol et 3 mmol (1eq.) d'éther d'énol préalablement porté à 0°C, on ajoute 5 ml d'acide formique et 1 ml d'eau, préalablement refroidis à 0°C. On laisse réagir pendant 30 mn à 0°C, puis ajoute 5 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 93/7). Le rendement est de 45 % pour Z = SiMe₃ et 39% pour Z=Me.

### Accès au rétinal

On met en oeuvre la réaction suivante :

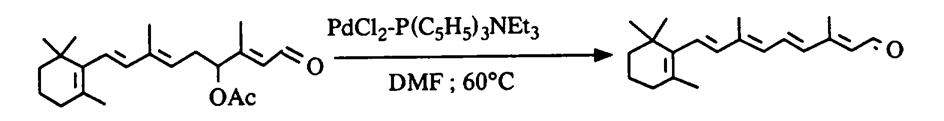

Le mode opératoire est identique à celui dans l'exemple 3. Le rendement est de 55 % en rétinal purifié par chromatographie éclair sur silice.

### Exemple 8 Procédé de préparation d'un dihydro rétinal par action d'un dérivé d'énol en C₅ sur le vinyl-β-ionol

On met en oeuvre la réaction suivante :

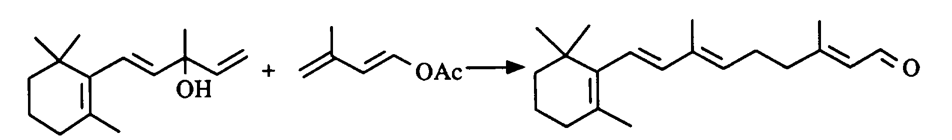

A un mélange de 660 mg (3 mmol) de vinyl-β-ionol et 380 mg d'acétate d'énol (3 mmol), on ajoute 5 ml d'acide formique et 1 ml d'eau. On laisse réagir pendant 60 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 47 %.

### Exemple 9 Procédé de préparation d'un dihydrorétinal par action d'un éther d'énol triméthylsilylé en C₅ sur le vinyl-β-ionol

On met en oeuvre la réaction suivante :

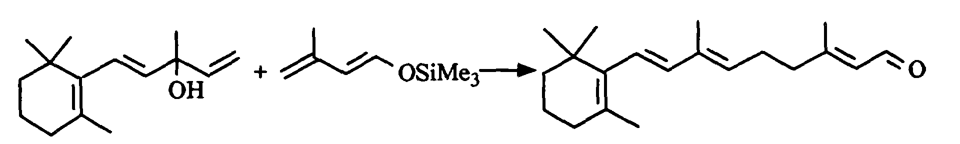

A un mélange de 660 mg (3 mmol) de vinyl-β-ionol et 470 mg d'éther d'énol (3 mmol) préalablement porté à -10°C, on ajoute 5 ml d'acide formique et 1 ml d'eau, préalablement refroidis à -10°C. On laisse réagir pendant 10 mn à -10°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4) : le rendement est de 82 %.

### Exemple 10 Procédé de préparation d'un dihydrorétinal par action d'un éther d'énol en C₇sur le β-ionol

On met en oeuvre la réaction suivante :

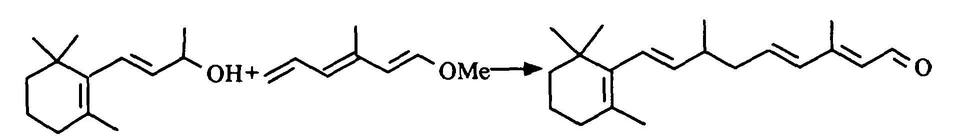

Sur une solution de 580 mg (3 mmol) de β-ionol et 370 mg (3 mmol) d'éther d'énol dans 6 ml de nitroéthane, refroidie à -20°C, on ajoute rapidement 190 µl d'éthérate de trifluorure de bore (soit 0.4 eq.), et laisse sous agitation à cette température pendant 1 h 30 mn. On hydrolyse par 5 ml d'une solution aqueuse saturée en hydrogénocarbonate de sodium : on extrait par 2 fois 5 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O=96/4). Le rendement est de 41 %.

### Exemple 11 Obtention d'un dérivé en C₁₈ de formule I(A=SPh) par action d'un éther d'énol en C ₃sur le vinyl-β-ionol

On met en oeuvre la réaction suivante :

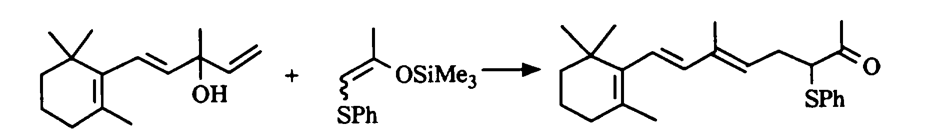

A un mélange de 660 mg (3 mmol) de vinyl-β-ionol et 710 mg d'éther d'énol (3 mmol) préalablement porté à 0°C, on ajoute 5 ml d'acide formique et 1 ml d'eau, préalablement refroidis à 0°C. On laisse réagir pendant 30 mn à 0°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 28 %.

### Accès à la cétone C₁₈

On met en oeuvre la réaction suivante :

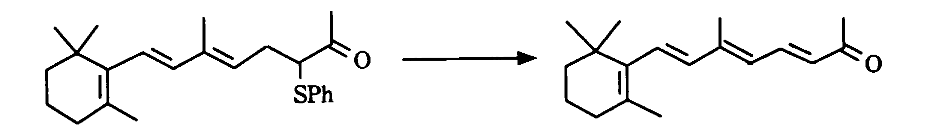

Le mode opératoire relatif à l'oxydation est le même que celui décrit à l'exemple 2. On part de 0.15 g de phénylthiocétone (0.41 mmol) et de l'acide métachloroperbenzoïque.

Le brut est repris dans 3 ml de tétrachlorure de carbone et la solution est chauffée à 60°C pendant 2 heures. On concentre et chromatographie sur silice pour obtenir 70 mg de cétone C₁₈ (Eluant : Eth. Pet./Et₂O = 96/4). Le rendement est de 67 %.

### Exemple 12 Obtention d'un dérivé en C₁₈ de formule I(A=Br) par action d'un éther d'énol en C₃ sur le vinyl-β-ionol

On met en oeuvre la réaction suivante :

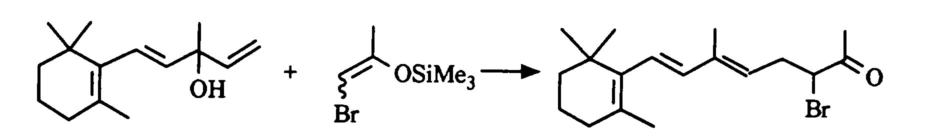

A un mélange de 660 mg (3mmol) de vinyl-β-ionol et 630 mg (3 mmol) d'éther d'énol préalablement porté à -10°C, on ajoute 5 ml d'acide formique et 1 ml d'eau, préalablement refroidis à -10°C. On laisse réagir pendant 30 mn à -10°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 54 %.

### Accès à la cétone C₁₈

On met en oeuvre la réaction suivante :

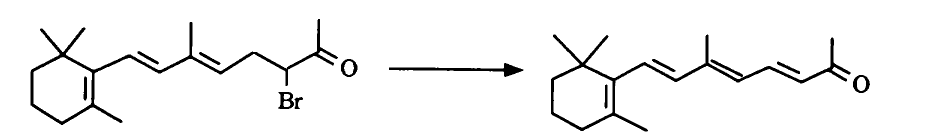

On place 0.5 g de C₁₈ bromée (1.47 mmol) dans 4 ml de dichlorométhane, puis ajoute 0.225 ml de DBU (1 eq.). On laisse sous agitation pendant 1 heure puis concentre et reprend par 5 ml d'éther, lave par 2 fois 3 ml d'eau, sèche sur sulfate de magnésium, filtre et concentre pour obtenir 220 mg de cétone C₁₈.

Le rendement est de 58 %.

### Exemple 13 Obtention d'un dérivé en C₁₈ de formule I(A=Cl) par action d'énol en C₃ sur le vinyl-β-ionol

On met en oeuvre la réaction suivante :

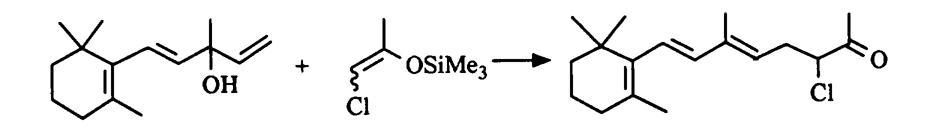

A un mélange de 660 mg (3 mmol) de vinyl-β-ionol et 500 mg (3 mmol) d'éther d'énol préalablement porté à -10°C, on ajoute 5 ml d'acide formique et 1 ml d'eau, préalablement refroidis à -10°C. On laisse réagir pendant 30 mn à -10°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. pet./Et₂O ) 96/4). Le rendement est de 54 %.

### Accès à la cétone C₁₈

On met en oeuvre la réaction suivante :

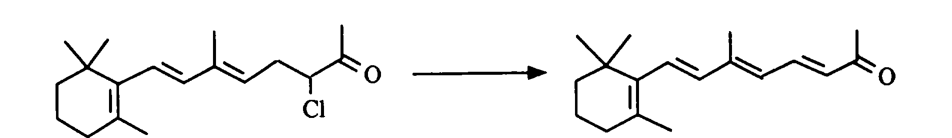

On place 0.45 g de C₁₈ chlorée (1.53 mmol) dans 3 ml de dichlorométhane, puis ajoute 0.235 ml de DBU (1 eq.). On laisse sous agitation pendant 15 heures puis concentre et reprend par 5 ml d'éther, lave par 2 fois 3 ml d'eau, sèche sur sulfate de magnésium, filtre et concentre pour obtenir 330 mg de cétone C₁₈. Le rendement est de 84 %.

### Exemple 14 Procédé de préparation d'un dérivé en C₁₈ par action d'un éther d'énol en C₃ sur le vinyl-β-ionol

On met en oeuvre la réaction suivante :

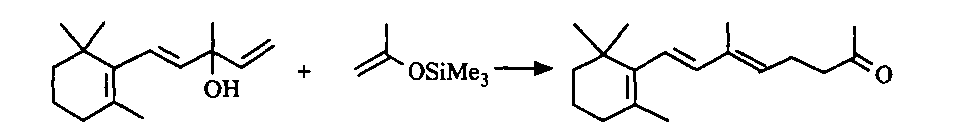

A un mélange de 660 mg (3 mmol) de vinyl-β-ionol et 390 mg (3 mmol) d'éther d'énol, on ajoute 5 ml d'acide formique et 1 ml d'eau. On laisse réagir pendant 30 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 42 %.

### Exemple 15 Obtention d'un dérivé en C₁₇ de formule I(A=Br) par action d'un bromo-éther d'énol en C₂ sur le vinyl-β-ionol

On met en oeuvre 1 réaction suivante :

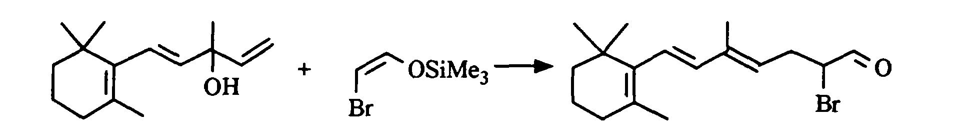

On place 0.66 g (3 mmol) de vinyl-β-ionol et 585 mg (1 eq.) de 1-bromo 2-triméthylsilyloxy éthylène dans 10 ml de nitroéthane, porte à -35°C, et ajoute 190 microlitres d'éthérate de trifluorure de bore. On laisse sous agitation pendant 1 heure à -35°C, puis hydrolyse par 6 ml d'une solution aqueuse d'hydrogénocarbonate de sodium, extrait au dichlorométhane.

Le produit attendu est caractérisé en RMN¹H.

RMN¹H (CDCl₃) : 9.5 (d, 1H, J = 6.4 Hz).

### Exemple 16 Obtention d'un dérivé en C₁₅ de formule I(A=SPh) par action d'un éther d'énol en C₂ sur le β-ionol

On met en oeuvre la réaction suivante :

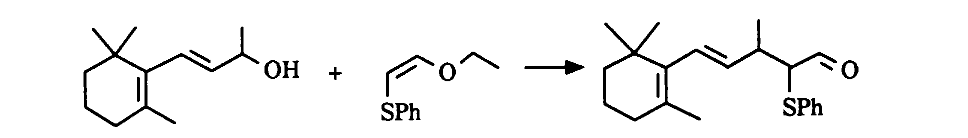

Sur une solution de 580 mg (3 mmol) de β-ionol et 500 mg (3 mmol) d'éther d'énol dans 6 ml de nitroéthane, refroidie à -20°C, on ajoute rapidement 190 µl d'éthérate de trifluorure de bore (soit 0.4 eq.), et laisse sous agitation à cette température pendant 1 h 30 mn. On hydrolyse par 5 ml d'une solution aqueuse saturée en hydrogénocarbonate de sodium : on extrait par 2 fois 5 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 63 %.

### Accès à l'aldéhyde C₁₅ (β ionylidèneacétaldéhyde)

On met en oeuvre la réaction suivante :

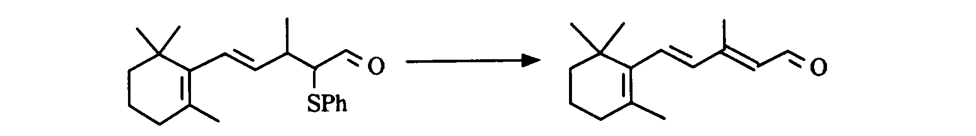

Le mode opératoire relatif à l'oxydation est le même que celui précédemment décrit à l'exemple 2. On part de 0.15 g d'aldéhyde précédent (0.46 mmole) et 87 mg (0.51 mmol ; 1.1 eq) d'acide métachloroperbenzoïque.

Le brut est repris dans 4 ml de tétrachlorure de carbone et la solution est chauffée à 60°C pendant 1 heure. On concentre et reprend par 3 ml d'éther, lave par de l'eau, sèche sur sulfate de magnésium, filtre et concentre : l'aldéhyde C₁₅ est détecté en RMN¹H.

### Exemple 17 Obtention d'un dérivé en C₁₅ de formule I(A=Br) par action d'un éther d'énol en C ₂ sur le β-ionol

On met en oeuvre la réaction suivante :

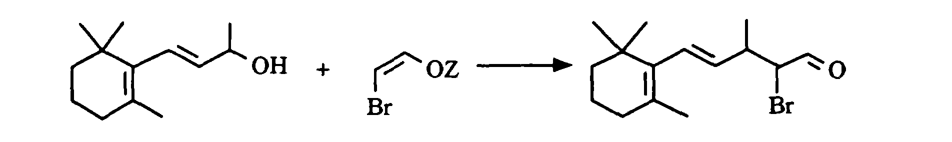

Z = Et ou SiMe₃

Sur une solution de 580 mg (3 mmol) de β-ionol et 3 mmol d'éther d'énol dans 6 ml de nitroéthane, refroidie à -20°C, on ajoute rapidement 190 µl d'éthérate de trifluorure de bore (soit 0.4 eq), et laisse sous agitation à cette température pendant 1 h 30 mn. On hydrolyse par 5 ml d'une solution aqueuse saturée en hydrogénocarbonate de sodium : on extrait par 2 fois 5 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4).

Le rendement est quantitatif dans le cas de l'éther d'énol triméthylsilylé et il est de 64 % dans le cas de l'éther d'énol d'éthyle.

### Accès à l'aldéhyde C₁₅ (β ionylidèneacétaldéhyde)

On met en oeuvre la réaction suivante :

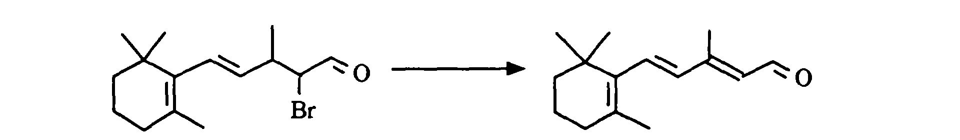

On place 0.9 g d'aldéhyde bromé (3 mmol) dans 8 ml de dichlorométhane, puis ajoute 0.46 ml de DBU (1 eq.). On laisse sous agitation pendant 1 heure puis concentre et reprend par 5 ml d'éther, lave par 2 fois 3 ml d'eau, sèche sur sulfate de magnésium, filtre et concentre. le produit brut est chromatographié sur silice (Eluant : Eth. Pet./Et₂O = 98/2), et on recueille 0,43 g d'aldéhyde C₁₅. Le rendement est de 66 %.

### Exemple 18 Obtention d'un dérivé en C₁₅ de formule II(A=Cl) par action d'un chloro-éther d'énol en C₅ sur un alcool en C₁₀

On met en oeuvre la réaction suivante :

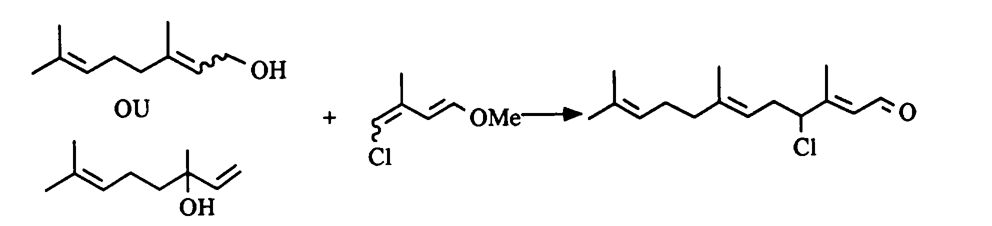

Dans 4.5 ml de nitroéthane, on introduit 400 mg (3 mmol) de chloro-éther d'énol en C₅ et 1 eq. d'isopropanol. On porte à -35°C, et ajoute 0.5 eq. de chlorure de zinc et 460 mg (3 mmol) de linalol (ou d'un mélange de géraniol et nérol).

Après 10 mn à -35°C, on hydrolyse par 6 ml d'une solution aqueuse d'hydrogénocarbonate de sodium, extrait au dichlorométhane.

Le produit attendu est caractérisé en RMN¹H.

RMN¹H(CDCl₃) : 10.05(d, 1H,J = 8 Hz).

### Exemple 19 Procédé de préparation d'un dihydro ψ rétinal par action d'un éther d'énol sur le vinyl-ψ-ionol

On met en oeuvre la réaction suivante :

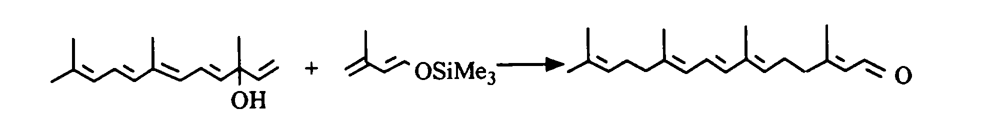

A un mélange de 660 mg (3 mmol) de vinyl-ψ-ionol et 470 mg (3 mmol) d'éther d'énol, on ajoute 5 ml d'acide formique et 1 ml d'eau. On laisse réagir pendant 30 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 72 %.

### Exemple 20 Obtention d'un dérivé en C₂₀ de formule II(A=Cl) par action d'un éther d'énol sur le vinyl-ψ-ionol

On met en oeuvre la réaction suivante :

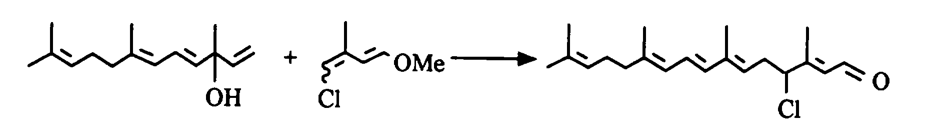

A un mélange de 660 mg (3 mmol) de vinyl-ψ-ionol et 400 mg (3 mmol) d'éther d'énol, on ajoute 5 ml d'acide formique et 1 ml d'eau. On laisse réagir pendant 30 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth/Pet./Et₂O = 96/4). Le rendement est de 63 %.

### Accès à l'aldéhyde C₂₀ insaturé

On met en oeuvre la réaction suivante :

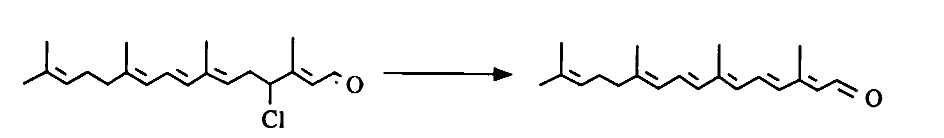

On place 0.23 g de C₂₀ chloré (0.71 mmol) dans 2 ml de dichlorométhane, puis ajoute 0,11 ml de DBU (1eq.). On laisse sous agitation pendant 2 heures puis concentre et reprend par 5 ml d'éther, lave par 2 fois 3 ml d'eau, sèche sur sulfate de magnésium, filtre et concentre pour obtenir 130 mg d'aldéhyde C₂₀ insaturé. Le rendement est de 64 %.

### Exemple 21 Obtention d'un dérivé en C₁₈ de formule II(A=Cl) par action d'un éther d'énol en C₃ sur le vinyl-ψ-ionol

On met en oeuvre la réaction suivante :

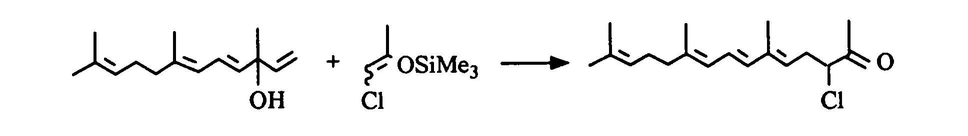

A un mélange de 660 mg (3 mmol) de vinyl-ψ-ionol et 500 mg (3 mmol) d'éther d'énol, on ajoute 5 ml d'acide formique et 1 ml d'eau. On laisse réagir pendant 30 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4).

### Accès à la cétone C₁₈

On met en oeuvre la réaction suivante :

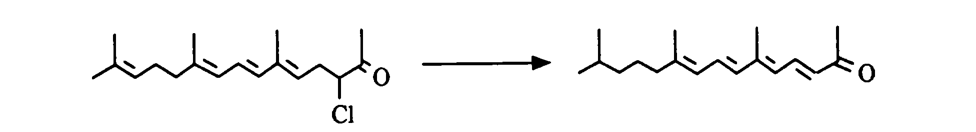

On place 0.41 g de C₁₈ chlorée (1.4 mmol) dans 3 ml de dichlorométhane, puis ajoute 0.21 ml de DBU (1 eq.). On laisse sous agitation pendant 15 heures puis concentre et reprend par 5 ml d'éther, lave par 2 fois 3 ml d'eau, sèche sur sulfate de magnésium, filtre et concentre pour obtenir après purification par chromatographie éclair sur silice (Eth. Pet/Eth = 96/4) 330 mg de ψ-phytone.

Le rendement est de 46 % par rapport au vinyl-ψ-ionol.

### Exemple 22 Procédé de préparation d'un dérivé en C₁₅ par action d'un éther d'énol en C₅ sur un alcool en C₁₀

On met en oeuvre la réaction suivante :

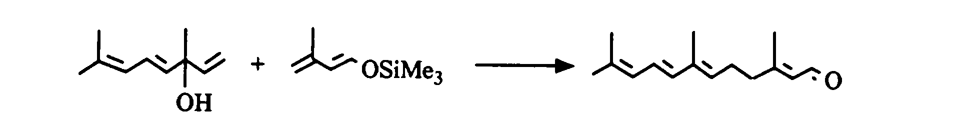

A un mélange de 456 mg (3 mmol) d'alcool en C₁₀ et 460 mg (3 mmol) d'éther d'énol, on ajoute 5 ml d'acide formique et 1 ml d'eau. On laisse réagir pendant 30 mn à 20°C, puis ajoute 3 ml de pentane et lave la phase pentane par 2 fois 1.5 ml d'eau. On sèche sur MgSO₄ puis filtre et concentre : le produit attendu est purifié par chromatographie éclair sur silice (Eluant : Eth. Pet./Et₂O = 96/4). Le rendement est de 65 %.

## Revendications

1. Nouveaux intermédiaires de la vitamine A répondant à la formule (I) suivante: 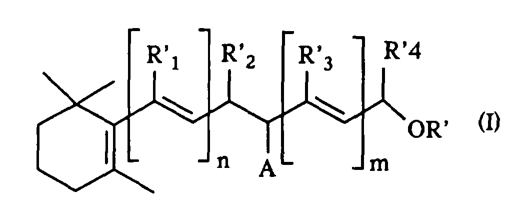 dans laquelle
- R'₁, R₂'_{,} R'₃ et R'₄ représentent chacun un motif semblable ou différent dans chacun des motifs insaturés choisi parmi l'hydrogène ou un motif alkyle,
- A représente un atome d'halogène choisi parmi le chlore ou le brome, un groupe arylthio ou arylséléno
- R' représente un atome d'hydrogène, une liaison avec l'atome de carbone de la chaîne, un groupe alkyle ou un groupe alkylcarbonyle
- n et m sont des nombres entiers égaux à 0, 1, 2 ou 3,
A n'étant pas un brome ou un chlore lorsque m est égal à 2 et n est égal à 1 et lorsque n = 0 et m = 3.

2. Composés intermédiaires des vitamines A et E et des caroténoïdes répondant à la formule générale (Il) suivante : 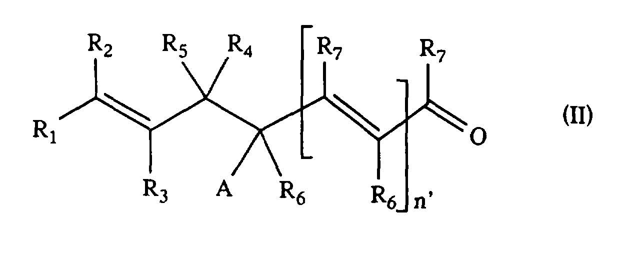 dans laquelle :
- R₁, R₂, R₃, R₄, R₅, R₆ et R₇ représentent chacun un groupe semblable ou différent choisi parmi les motifs hydrogène, alkyle, alkényle éventuellement cyclique deux des substituants pouvant former entre eux un cycle insaturé ou saturé,
- A représente un halogène choisi parmi le chlore et le brome, un groupe arylthio ou arylséléno,
- n' est un nombre entier compris entre 0 et 10.

3. Nouveaux intermédiaires selon les revendications 1 et 2 caractérisés en ce que A représente le chlore.

4. Nouveaux intermédiaires selon les revendications 1 et 3 caractérisés en ce que A représente le chlore et n+m est égal à 3.

5. Procédé de préparation de composés de formules (I) et (II) caractérisé en ce que l'on fait réagir un composé de formule (III) suivante: 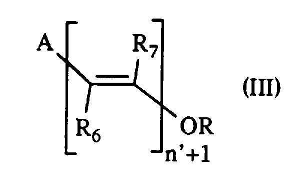 dans laquelle:
- R représente un groupe alkyle ou silyle ayant un à quatre substituants carbonés, un groupe alkylcarbonyle ou tosyle,
- R₇ et R₆ représentent chacun, dans chacun des motifs insaturés, un substituant identique ou différent choisi parmi l'hydrogène ou un groupe alkyle ayant un à quatre atomes de carbone,
- A représente l'hydrogène, un atome d'halogène choisi parmi le chlore ou le brome, un groupe arylthio, arylséléno, acyloxy, trialkylsilyle,
- n' représente un nombre entier compris entre 0 et 10,
avec un composé de formule (IV) suivante: 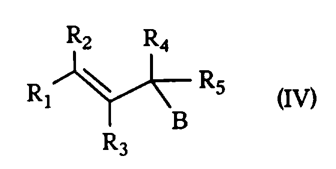 dans laquelle :
- R₁, R₂, R₃, R₄ et R₅ représentent l'hydrogène, un motif alkyle ayant un à vingt atomes de carbone, un motif alkényle ayant deux à vingt atomes ou peuvent former entre eux une chaîne terpénique polyénique, alkylène ou alkénylène cyclique éventuellement substituée,
- B représente un groupe hydroxyle, alcoxyle ayant 1 à 6 atomes de carbone, alcoyle, aryloxy ou halogène.

6. Procédé selon la revendication 5 caractérisé en ce que les dérivés de formule (IV) ayant 10 atomes de carbone répondent aux formules suivantes:

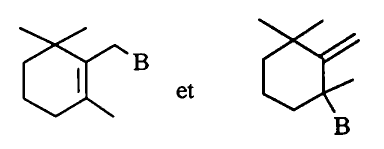

7. Procédé selon la revendication 5 caractérisé en ce que les dérivés de formule (IV) ayant 13 atomes de carbone répondent aux formules suivantes:

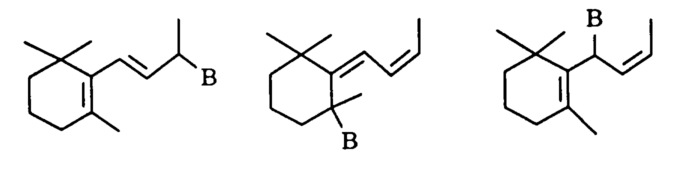

8. Procédé selon la revendication 5 caractérisé en ce que les dérivés de formule (IV) ayant 15 atomes de carbone répondent aux formules suivantes:

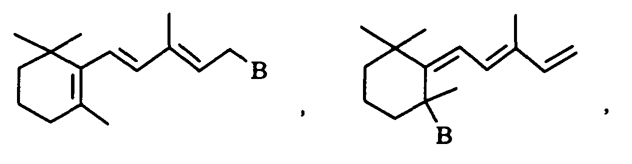

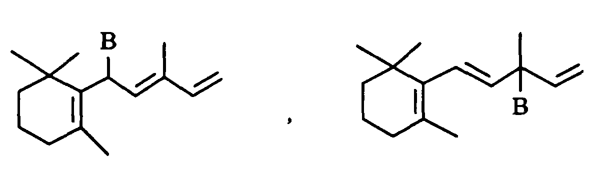

9. Procédé selon la revendication 5 caractérisé en ce que les dérivés de formule (IV) ayant 18 atomes de carbone répondent aux formules suivantes:

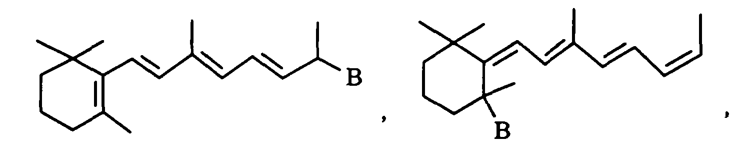

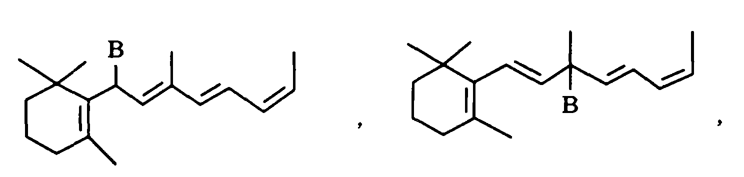

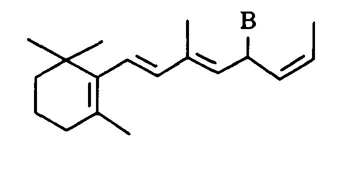

10. Procédé de préparation de composés de formule (III) caractérisé en ce qu'on effectue un craquage thermique des acétals de formule (V) suivante : 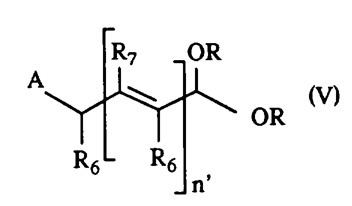 dans laquelle A, R₇, R₆, R et n' ont la même signification que précédemment

11. Procédé selon l'une quelconque des revendications 5 à 9 caractérisé en ce qu'on effectue la condensation du composé de formule (III) avec celui de formule (IV) en présence d'un acide de Lewis ou protique.

12. Procédé selon la revendication 11 caractérisé en ce que l'acide de Lewis ou protique est un acide choisi parmi le chlorure de zinc, l'éthérate de trifluorure de bore, le chlorure ferrique, l'acide trichloroactique, l'acide trifluoroacétique, le triflate de triméthylsilyle, les chlorures stanneux ou stannique, l'acide formique, l'acide trifluorométhane sulfonique, le triflate de diméthyl tertiobutylsilyle, le trichlorure de bismuth, le tétrachlorure de titane, les catalyseurs hétérogènes acides.

13. Procédé selon la revendication 12 caractérisé en ce que la condensation est effectuée en présence d'un alcool et/ou d'eau.

14. Procédé selon l'une quelconque des revendications 12 et 13 caractérisé en ce que la condensation est effectuée dans un solvant choisi parmi les nitriles, les nitroalkanes, les nitroaryles, les solvants aliphatiques ou aromatiques halogénés, les sulfones ou les acides organiques.

15. Procédé selon l'une quelconque des revendications 12 à 14 caractérisé en ce que la température du milieu réactionnel est comprise entre -45°C et 50°C.

16. Procédé de préparation du rétinal caractérisé en ce que, dans une première étape, l'on prépare un composé de formule (I) en C20 selon la revendication 1 par le procédé selon l'une quelconque des revendications 5 à 9 et 11 à 15 et, dans une deuxième étape, on élimine le groupe A par l'action d'une base.

17. Procédé selon la revendication 16 caractérisé en ce que la base est choisie parmi le diazabicyclooctane, le diazabicyclononène, le chlorure de lithium dans un solvant aprotique polaire, la quinuclidine, la potasse dans un hydrocarbure ou le palladium en présence d'une base.

## Patentansprüche

1. Neue Zwischenprodukte für Vitamin A, die der folgenden Formel (I) entsprechen: 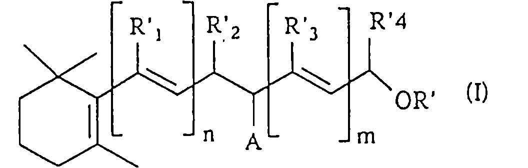 in der
- R'₁, R'₂, R'₃ und R'₄ jeweils eine gleiche oder verschiedene Einheit in jeder der ungesättigten Einheiten darstellen, die aus Wasserstoff oder einer Alkyl-Einheit ausgewählt ist,
- A ein Halogenatom, das aus Chlor oder Brom ausgewählt ist, eine Arylthio- oder Arylseleno-Gruppe darstellt,
- R' ein Wasserstoffatom, eine Bindung mit dem Kohlenstoffatom der Kette, eine Alkyl-Gruppe oder eine Alkylcarbonyl-Gruppe darstellt,
- n und m ganze Zahlen gleich 0, 1, 2 oder 3 sind,
wobei A nicht Brom oder Chlor ist, wenn m gleich 2 ist und n gleich 1 ist und wenn n = 0 und m = 3.

2. Zwischenprodukte für die Vitamine A und E und die Carotinoide, die der folgenden allgemeinen Formel (II) entsprechen: 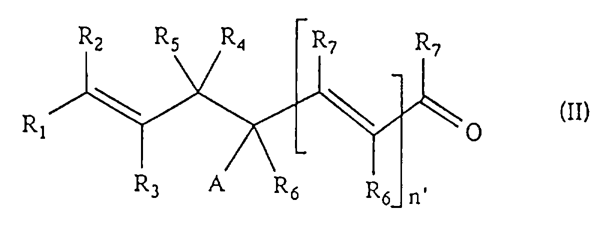 in der
- R₁, R₂, R₃, R₄, R₅, R₆ und R₇ jeweils eine gleiche oder verschiedene Gruppe darstellen, die ausgewählt ist aus den Einheiten Wasserstoff, Alkyl, Alkenyl, gegebenenfalls cyclisch, wobei zwei der Substituenten unter sich einen ungesättigten oder gesättigten Ring bilden können,
- A ein Halogenatom, das aus Chlor und Brom ausgewählt ist, eine Arylthio- oder Arylseleno-Gruppe darstellt,
- n' eine ganze Zahl zwischen 0 und 10 eingeschlossen ist.

3. Neue Zwischenprodukte nach den Ansprüchen 1 und 2, dadurch gekennzeichnet, daß A Chlor darstellt.

4. Neue Zwischenprodukte nach den Ansprüchen 1 und 3, dadurch gekennzeichnet, daß A Chlor darstellt und n+m gleich 3 ist.

5. Verfahren zur Herstellung von Verbindungen der Formeln (I) und (II), dadurch gekennzeichnet, daß man eine Verbindung der folgenden Formel (III): 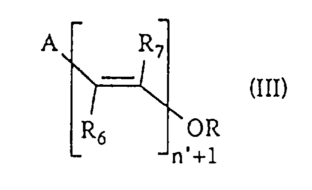 in der:
- R eine Alkyl- oder Silyl-Gruppe mit 1 bis 4 Kohlenstoff-Substituenten, eine Alkylcarbonyl- oder Tosyl-Gruppe darstellt,
- R₇ und R₆ jeweils in jeder der ungesättigten Einheiten einen identischen oder verschiedenen Substituenten darstellen, der aus Wasserstoff oder einer Alkyl-Gruppe mit 1 bis 4 Kohlenstoffatomen ausgewählt ist,
- A ein Wasserstoffatom, ein Halogenatom, ausgewählt aus Chlor oder Brom, eine Arylthio-, Arylseleno-, Acyloxy-, Trialkylsilyl-Gruppe darstellt,
- n' eine ganze Zahl zwischen 0 und 10 eingeschlossen darstellt,
mit einer Verbindung der folgenden Formel (IV) reagieren läßt: 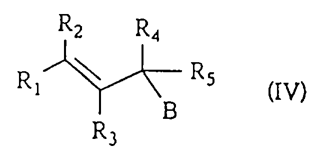 in der
- R₁, R₂, R₃, R₄ und R₅ Wasserstoff, eine Alkyleinheit mit 1 bis20 Kohlenstoffatomen, eine Alkenyleinheit mit 2 bis 20 Kohlenstoffatomen darstellen oder unter sich eine gegebenenfalls substituierte cyclische terpenische Polyen-, Alkylen- oder Alkenylenkette bilden können,
- B eine Hydroxyl-, Alkoxylgruppe mit 1 bis 6 Kohlenstoffatomen, Alkyl-, Aryloxygruppe oder Halogen darstellt.

6. Verfahren nach Anspruch 5, dadurch gekennzeichnet, daß die Derivate der Formel (IV) mit 10 Kohlenstoffatomen den folgenden Formeln entsprechen:

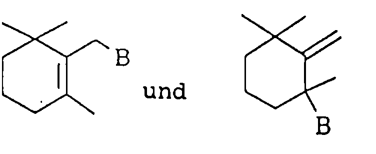

7. Verfahren nach Anspruch 5, dadurch gekennzeichnet, daß die Derivate der Formel (IV) mit 13 Kohlenstoffatomen den folgenden Formeln entsprechen:

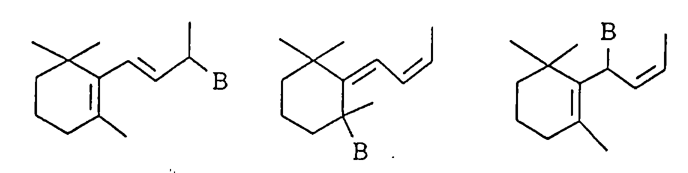

8. Verfahren nach Anspruch 5, dadurch gekennzeichnet, daß die berivate der Formel (IV) mit 15 Kohlenstoffatomen den folgenden Formeln entsprechen:

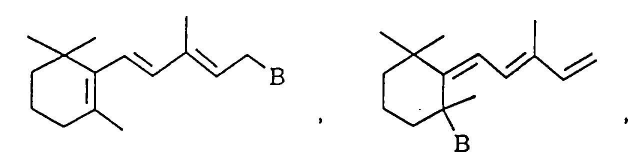

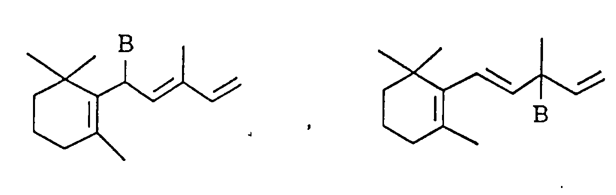

9. Verfahren nach Anspruch 5, dadurch gekennzeichnet, daß die Derivate der Formel (IV) mit 18 Kohlenstoffatomen den folgenden Formeln entsprechen:

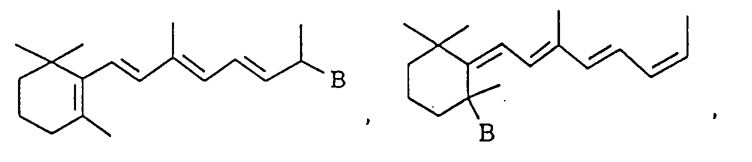

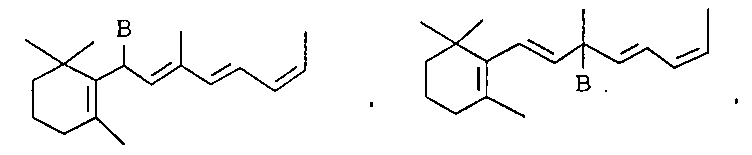

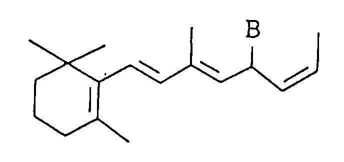

10. Verfahren zur Herstellung von Verbindungen der Formel (III), dadurch gekennzeichnet, daß man ein thermisches Cracken der Acetale der folgenden Formel (V) bewirkt: 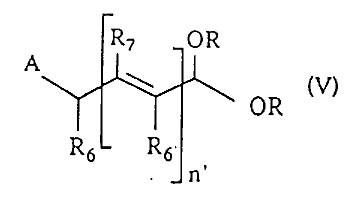 in der jedes von A, R₇, R₆, R und n' die gleiche Bedeutung wie vorstehend aufweist.

11. Verfahren nach irgendeinem der Ansprüche 5 bis 9, dadurch gekennzeichnet, daß man die Kondensation der Verbindung der Formel (III) mit derjenigen der Formel (IV) in Gegenwart einer Lewis- oder protischen Säure vornimmt.

12. Verfahren nach Anspruch 11, dadurch gekennzeichnet, daß die Lewis- oder protische Säure eine Säure ist, die aus Zinkchlorid, Bortrifluoridetherat , Eisen(III)chlorid, Trichloressigsäure, Trifluoressigsäure, Trimethylsilyltriflat, Zinn(II)- oder Zinn(IV)chlorid, Ameisensäure, Trifluormethansulfonsäure, Dimethyl-tert-butylsilyltriflat, Wismuttrichlorid, Titantetrachlorid, heterogenen sauren Katalysatoren ausgewählt ist.

13. Verfahren nach Anspruch 12, dadurch gekennzeichnet, daß die Kondensation in Gegenwart eines Alkohols und/oder von Wasser vorgenommen wird.

14. Verfahren nach irgendeinem der Ansprüche 12 und 13, dadurch gekennzeichnet, daß die Kondensation in einem Lösungsmittel vorgenommen wird, das aus Nitrilen, Nitroalkanen, Nitroatylen, aliphatischen oder aromatischen halogenierten Lösungsmitteln, Sulfonen oder organischen Säuren ausgewählt ist.

15. Verfahren nach irgendeinem der Ansprüche 12 bis 14, dadurch gekennzeichnet, daß die Temperatur des Reaktionsmediums zwischen -45°C und 50°C eingeschlossen ist.

16. Verfahren zur Herstellung von Retinal, dadurch gekennzeichnet, daß man in einem ersten Schritt eine C20-Verbindung der Formel (I) nach Anspruch 1 durch das Verfahren nach irgendeinem der Ansprüche 5 bis 9 und 11 bis 15 herstellt und in einem zweiten Schritt durch die Einwirkung einer Base die Gruppe A entfernt.

17. Verfahren nach Anspruch 16, dadurch gekennzeichnet, daß die Base aus Diazabicyclooctan, Diazabicyclononen, Lithiumchlorid in einem aprotischen polaren Lösungsmittel, Chinuclidin, Kaliumhydroxid in einem Kohlenwasserstoff oder Palladium in Gegenwart einer Base ausgewählt wird.

## Claims

1. New intermediates of vitamin A, of the following formula (I): 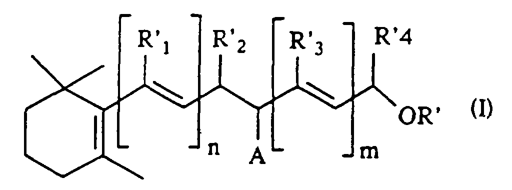 in which
- R'₁, R'₂, R'₃ and R'₄ each represent a similar or different unit, in each of the unsaturated units, which is chosen from hydrogen or an alkyl unit,
- A represents a halogen atom chosen from chlorine or bromine, an arylthio or an arylseleno group,
- R' represents a hydrogen atom, a bond with the carbon atom of the chain, an alkyl group or an alkylcarbonyl group,
- n and m are integers equal to 0, 1, 2 or 3, A not being bromine or chlorine when m is equal to 2 and n is equal to 1, and when n = 0 and m = 3.

2. Intermediate compounds of vitamins A and E and of carotenoids, of the following general formula (II): 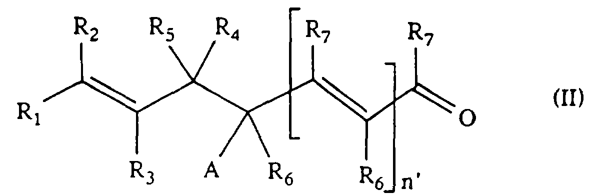 in which:
- R₁, R₂, R₃, R₄, R₅, R₆ and R₇ each represent a similar or different group which is chosen from hydrogen, alkyl or optionally cyclic alkenyl units, it being possible for two of the substituents to form an unsaturated or saturated ring with one another,
- A represents a halogen chosen from chlorine and bromine, an arylthio or arylseleno group,
- n' is an integer between 0 and 10.

3. New intermediates according to claims 1 and 2, characterised in that A represents chlorine.

4. New intermediates according to claims 1 and 3, characterised in that A represents chlorine and n+m is equal to 3.

5. Process for preparing the compounds of formulae (I) and (II), characterised in that a compound of the following formula (III): 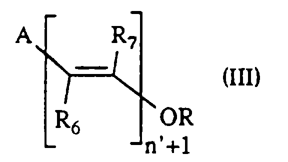 in which:
- R represents an alkyl or silyl group having one to four carbon substituents, an alkylcarbonyl or a tosyl group,
- R₇ and R₆ each represent, in each of the unsaturated units, an identical or different substituent chosen from hydrogen or an alkyl group having one to four carbon atoms,
- A represents hydrogen, a halogen atom chosen from chlorine or bromine, an arylthio, arylseleno, acyloxy or trialkylsilyl group,
- n' represents an integer between 0 and 10,
is reacted with a compound of the following formula (IV): 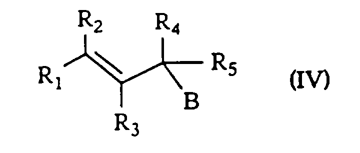 in which:
- R₁, R₂, R₃, R₄ and R₅ represent hydrogen, an alkyl unit having one to twenty carbon atoms, an alkenyl unit having two to twenty carbon atoms, or they may form with one another an optionally substituted cyclic polyene terpene, alkylene or alkenylene chain,
- B represents a hydroxyl group or an alkoxy group having 1 to 6 carbon atoms, an alkyl or aryloxy group or a halogen.

6. Process according to claim 5, characterised in that the derivatives of formula (IV) having 10 carbon atoms are of the following formulae:

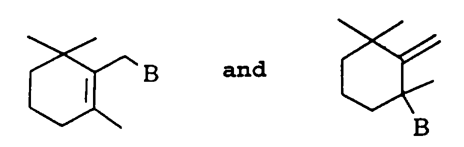

7. Process according to claim 5, characterised in that the derivatives of formula (IV) having 13 carbon atoms are of the following formulae:

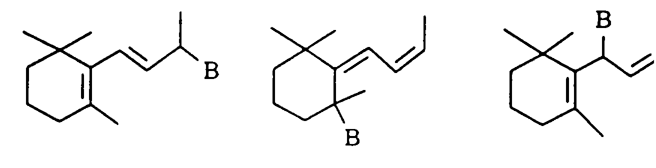

8. Process according to claim 5, characterised in that the derivatives of formula (IV) having 15 carbon atoms are of the following formulae:

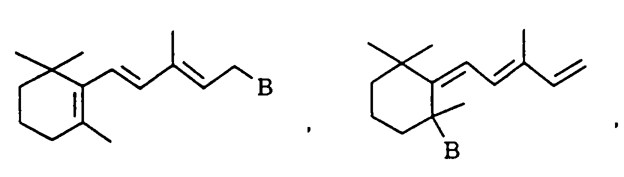

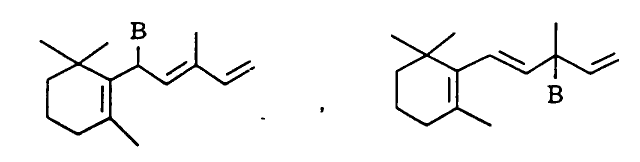

9. Process according to claim 5, characterised in that the derivatives of formula (IV) having 18 carbon atoms are of the following formulae:

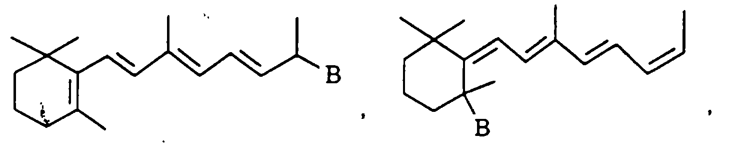

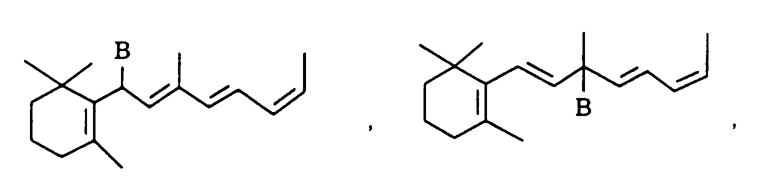

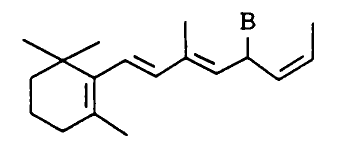

10. Process for preparing the compounds of formula (III), characterised in that a thermal cracking is carried out of the acetals of the following formula (V): 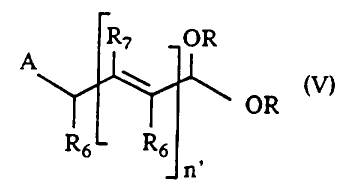 in which A, R₇, R₆, R and n' have the same meaning as above.

11. Process according to any one of claims 5 to 9, characterised in that the condensation of the compound of formula (III) with that of formula (IV) is carried out in the presence of a Lewis or a protic acid.

12. Process according to claim 11, characterised in that the Lewis or protic acid is an acid chosen from zinc chloride, boron trifluoride etherate, ferric chloride, trichloroacetic acid, trifluoroacetic acid, trimethylsilyl triflate, stannous or stannic chlorides, formic acid, trifluoromethanesulphonic acid, dimethyl tert-butylsilyl triflate, bismuth trichloride, titanium tetrachloride and heterogeneous acid catalysts.

13. Process according to claim 12, characterised in that the condensation is carried out in the presence of an alcohol and/or water.

14. Process according to either of claims 12 and 13, characterised in that the condensation is carried out in a solvent chosen from nitriles, nitroalkanes, nitroaryls, halogenated aliphatic or aromatic solvents, sulphones or organic acids.

15. Process according to any one of claims 12 to 14, characterised in that the temperature of the reaction medium is between -45°C and 50°C.

16. Process for preparing retinal, characterised in that, in a first step, a C₂₀ compound of Formula (I) according to claim 1 is prepared using the process according to any one of claims 5 to 9 and 11 to 15 and, in a second step, the A group is removed by the action of a base.

17. Process according to claim 16, characterised in that the base is chosen from diazabicyclooctane, diazabicyclononene, lithium chloride in a polar aprotic solvent, quinuclidine, potassium hydroxide in a hydrocarbon or palladium in the presence of a base.
